# EUROPEAN PATENT SPECIFICATION

(11) **EP 2 250 976 B1**
(45) Date of publication and mention of the grant of the patent: **26.08.2015**
(21) Application number: 09160186.4
(22) Date of filing: 13.05.2009
(51) Int. Cl.: A61F 2/95, A61F 2/24

(54) **Device for the in situ delivery of heart valves**
Vorrichtung zur In-situ-Lieferung von Herzklappen
Dispositif pour la livraison in situ de valvules cardiaques

(43) Date of publication of application: 17.11.2010
(73) Proprietor: Sorin Group Italia S.r.l., 13040 Saluggia (VC) (IT)
(72) Inventor: Giannetti, Arnaldo, 13044, Crescentino (Vercelli) (IT); Ghione, Laura, 10131, Torino (IT); Gaschino, Paolo, 10090, Castagneto Po (Torino) (IT); Righini, Giovanni, 10034, Chivasso (Torino) (IT)
(74) Representative: Bosotti, Luciano

(56) References cited:
- WO-A-2006/138173
- US-A1- 2001 044 591
- US-A1- 2007 203 561

## Description

### TECHNICAL FIELD

The present invention relates to devices for the in situ delivery of heart valves. More specifically, the invention relates to delivery devices for cardiac valve prostheses using minimally-invasive surgical techniques or endovascular delivery techniques. Such devices are known, for example, from US-A-2001/0044591, WO-A-2006/0138173 and US-A-2007/0203561.

### BACKGROUND

Expandable prosthetic valves typically include an expandable and collapsible anchoring structure or armature, which is able to support and fix the valve prosthesis in the implantation position, and prosthetic valve elements, generally in the form of leaflets or flaps, which are stably connected to the anchoring structure and are able to regulate blood flow.

These expandable prosthetic valves enable implantation using various minimally invasive or sutureless techniques. Exemplary applications for such an expandable valve prosthesis include aortic and pulmonary valve replacement. Various techniques are generally known for implanting an aortic valve prosthesis and include percutaneous implantation (e.g., transvascular delivery), dissection of the ascending aorta using minimally invasive thoracic access (e.g., mini-thoracotomy or ministemotomy), and transapical delivery wherein the aortic valve annulus is accessed through an opening near the apex of the left ventricle. The percutaneous and thoracic access approaches involve delivering the prosthesis in a direction opposing blood flow (i.e., retrograde), whereas the transapical approach involves delivering the prosthesis in the same direction as blood flow (i.e., antegrade).

### SUMMARY

The present invention, according to an embodiment, is a device for delivering a cardiac valve prosthesis to an implantation site, which includes a distal valve holder portion and a shaft coupled to the valve holder portion.

The shaft is selectively bendable to a curved shape to selectively vary the spatial orientation of the valve holder portion with respect to the implantation site.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

Figure 1 is a general perspective view of a valve delivery device according to an exemplary embodiment.

Figures 2a and 2b are longitudinal sectional views of the device of figure 1 according to exemplary embodiments. Figures 2a and 2b show an exploded view, wherein the components shown in figure 2b are intended to be located within the components shown in figure 2a.

Figure 3 is a partial sectional view of some of a portion of the device shown in figure 2.

Figure 4 is a cross-sectional view taken along line IV-IV of figure 3.

Figure 5 is a cross-sectional view taken along line V-V of figure 3.

Figure 6 is a perspective view showing a portion of the device indicated by an arrow VI in figure 2b.

Figure 7 is a perspective view showing a portion of the device indicated by an arrow VII in figure 2b.

Figure 8 is a perspective view of a valve delivery device according to another exemplary embodiment.

Figure 9 is a perspective view of an exemplary component of an embodiment.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

In the following description, numerous specific details are given to provide a thorough understanding of embodiments. The embodiments can be practiced without one or more of the specific details, or with other methods, components, materials, etc. Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

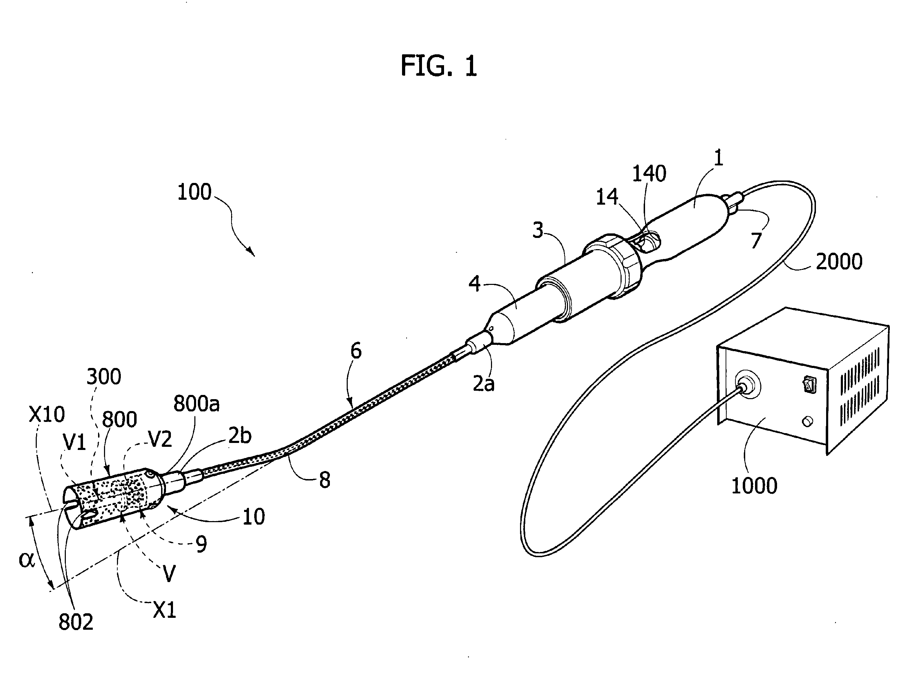

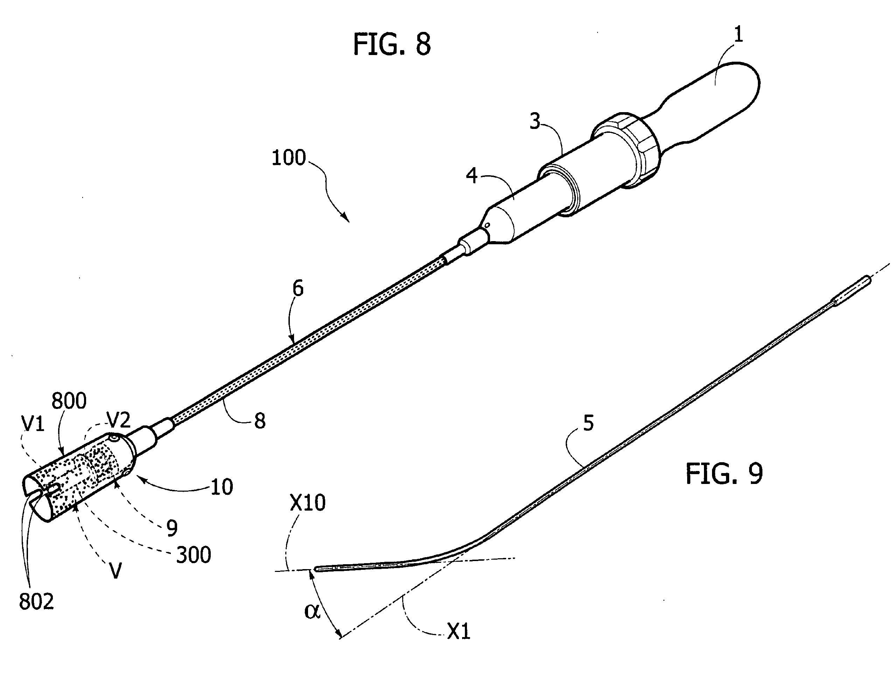

Figures 1 and 8 are perspective views of exemplary embodiments of a valve delivery device 100. The device 100 includes a handle 1 for manipulation by a practitioner and a holder unit 10 for a valve V to be delivered. As shown, the handle 1 and the holder unit 10 are generally located at proximal and distal ends of the device 100.

As used herein, "proximal" and "distal" refer to the conditions of handling of the device 100 by a practitioner who manipulates the device via the handle 1 at the "proximal" end in order to permit delivery of the valve V at the "distal" end of the device 100. Thus "proximal" and "distal," as used herein, have no direct relationship to the approach (retrograde or antegrade) adopted for delivering the valve V.

In one exemplary embodiment, the valve V is of the type disclosed in U.S. Publication 2006/0178740. Such a prosthetic valve includes two annular end portions V1, V2 (i.e. inflow and outflow with respect to the direction of unimpeded flow of blood through the valve).

As shown in figure 1, the valve is arranged in the holder unit 10 at the distal delivery end of the device 100 with the annular portions V1, V2 in a radially contracted condition.

In the exemplary illustrated arrangement, the annular portions V1 and V2 are located "distally" and "proximally," respectively of each other with reference to the orientation of the device 100. In the following it will be assumed that the valve V is delivered by releasing the annular portion V1 first and then by causing the valve V to gradually expand (e.g. due to its elastic or superelastic nature), starting from the portion V1 and continuing to the portion V2, until expansion is complete.

As further shown in figure 1, the device 100 includes a shaft 6, which is adapted to be selectively shaped into a curved pattern as further described below. The shaft 6 extends from the handle 1 to the holder unit 10 for the valve.

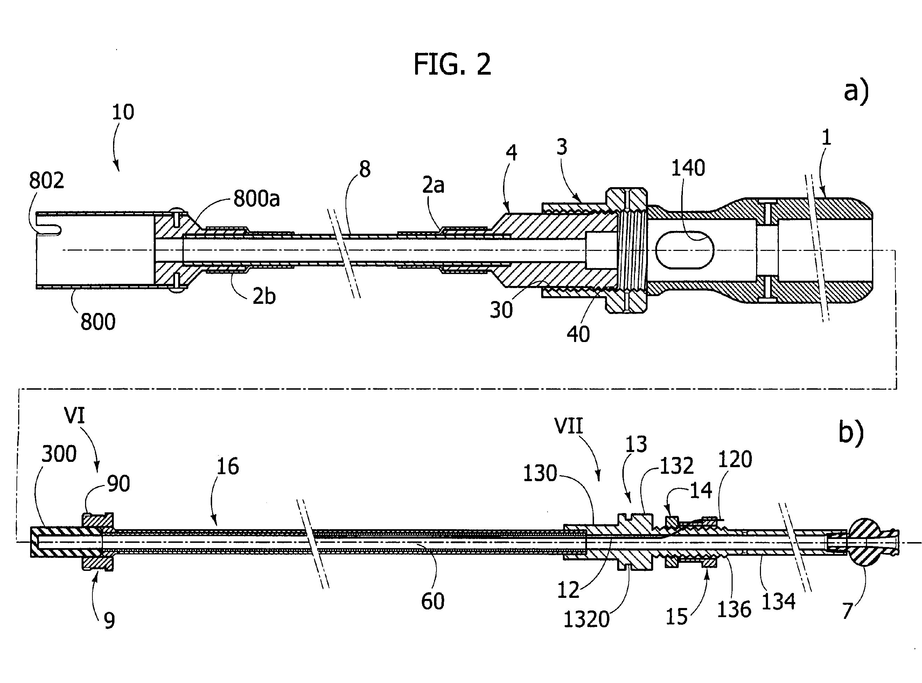

In various embodiments, the holder unit 10 includes an inner body or valve support 9 integral with or coupled to the tubular core 16 and including an annular groove or similar recessed 90 formation (see Figures 2b) adapted to receive the (proximal) annular portion V2 of the valve V in a radially contracted condition.

In the embodiments shown in figures 2 to 7, the shaft 6 includes a tubular sheath or sleeve 8 slidably arranged over the tubular core 16. The sleeve 8 is adapted to couple with or fit into a proximal sleeve 4, fixed in rotation with respect to the handle 1. The sleeve 4 has an outer threaded surface 40 to cooperate with a complementary threaded formation 30 provided at the inner surface of a tubular rotary actuation member 3 arranged around the sleeve 4. The actuation member 3 is fixed in translation with respect to the shaft 6. In an embodiment, a tapered sheath 2a acts as an interface between the proximal sleeve 4 and the sleeve 8.

The sleeve 8 extends over the tubular core 16 and terminates with a distal portion including a terminal enlarged portion 800 adapted to extend around the distal portion of the core 16 to form an external tubular member of the holder unit 10, which is adapted to radially constrain and retain the valve V when disposed therein.

The terminal enlarged portion 800 may be either one-piece with the rest of the sleeve 8 or, as shown in figure 2a, may include a separate tubular member coupled (e.g., adhesively or by means of screws, rivets, protrusions, etc.) to a funnel-shaped formation 800a located at the terminal end of the distal portion 80 of the sleeve 8. In an embodiment, a tapered sheath 2b acts as an interface between the sleeve 8 and the funnel shaped element 800a.

According to various embodiments, the threaded surface/formations 30, 40 comprise a "micrometric" device actuatable by rotating the actuation member 3 to produce and precisely control axial displacement of the sleeve 8, 800 over the core 16. Such a controlled movement may take place along the core 16 starting from an extended position, as shown in figure 1, where the outer member 800 of the holder unit 10 radially constrains and retains the valve V.

As the sleeves 4, 8 are gradually retracted towards the handle 1 (by operation of the actuation device 30, 40, which are controlled by the rotary member 3), the outer member 800 gradually releases first the annular portion V1 of the valve V, then the portion of the valve located between the annular portion V1 and the annular portion V2, and finally the annular portion V2 of the valve V, thus permitting gradual radial expansion of the valve V.

In an exemplary delivery procedure of the valve V, the practitioner introduces the device 100 into the patient's body and advances it through the delivery route or path until the outer member 800 is located at the annulus of the natural valve to be substituted by the valve V. The practitioner may use any of a variety of known techniques for delivering the device 100 to the valve annulus site.

In various embodiments, the radial dimensions of portion 800 are slightly less than the radial dimensions of the annulus of the natural valve intended to be substituted. In these embodiments, the outer member 800 will not unduly move about or "dance," while being positioned within the natural annulus. In various exemplary embodiments, these radial dimensions are in the range of between about 10 mm and about 27 mm.

In the exemplary case of aortic valve replacement, this may involve the outer member 800 being located immediately distally (with respect to the flow direction blood pumped from the left heart ventricle) of the aortic annulus so that the annular portions V1 and V2 are located on opposite sides (i.e. astride) of the Valsalva sinuses. In other words, the portion V1 is located on one of the ventricle side and the aortic root side of the Valsalva sinuses, and the portion V2 is located on the opposite side of the Valsalva sinuses.

Once the portion 800 is disposed properly at the annulus site, the practitioner will actuate the rotary actuation member 30 by rotating it in such a way that cooperation of the threaded sections 30 and 40 will cause the outer sleeve 8 and the proximal sleeve 4 to start gradually retracting towards the handle 1. As a result of this retraction of the outer sleeve, the outer member 800 will gradually disengage the annular portion V1 of the valve V. The annular portion V1 will thus be allowed to radially expand.

Gradual withdrawal of the sleeves 4, 8 proceeds until the outer member 800 has almost completely disengaged the valve V, while the annular formation V2 is still securely retained by the tubular member 800 which still forces the annular formation V2 of the valve within the inner body 9 of the holder portion.

This deployment mechanism of the annular formation V1 and the valve V may be controlled very precisely by the practitioner via the screw-like mechanism 30, 40 actuated by the rotary member 3. Deployment may take place in a gradual and easily controllable manner by enabling the practitioner to verify how deployment proceeds.

Also, so long as the annular formation V2 of the valve V is still constrained within the formation 9 by the tubular member 800, the practitioner still retains firm control of the partially (e.g., "basket-like") expanded valve V. The practitioner will thus be able to adjust the position of the valve V both axially and radially, that is by rotating the valve V around its longitudinal axis, e.g. to ensure that radially expanding anchoring formations of the valve V are precisely aligned with the Valsalva sinuses to firmly and reliably retain in place the valve V once finally delivered.

In various embodiments, the portion 800 has a marginal outer edge provided with one or more notches 802 providing a reference in angular positioning of the valve V at the implantation site. In various embodiments, these notches are visible during implantation (e.g., using radiography or other common implantation techniques).

According to various embodiment, the annular portion V2 of the valve V is received in the formation 9 and is thus blocked against any significant axial movement, during the retraction of the sleeve 8 and the sleeve 4 over the core 16. In other words, the valve V will not experience any significant axial displacement with respect to the shaft 6. The retraction of the outer sleeve 8 continues until the annular formation V2 (and the valve V as a whole) become disengaged from the device 100 and thus completely deployed at the implantation site.

While a cardiac valve prosthesis including two self-expandable annular portions has been considered herein for exemplary purposes, this disclosure similarly applied to cardiac valve prostheses including further expandable annular portions and/or one or more annular portions that are expandable via an expansion means such as an inflatable balloon.

In various embodiments, the device 100 includes an illuminator device 300 located at the holder unit 10 to provide illumination of the implantation site of the valve V. In minimally-invasive surgical procedures the operation site is observed directly by the practitioner via the (minimally-invasive) access path gained through the thorax of the patient. The action of the illuminator 300 is beneficial in that penetration of ambience light to the implantation site may be reduced or impeded by the body structures of the patient. In various embodiments, the illuminator device 300 is adjustable.

In the exemplary embodiment shown in figure 1, the illuminator 300 is fed with light radiation produced by a source 1000 via fiber optical element 2000 which extends through the shaft 6 (for instance by extending in an axial cavity 60 provided in the tubular core 16). Preferably (see also figure 2b), the fiber optical element 2000 enters the shaft 6 by means of a connector 7 (e.g, a Luer-Lock female connector). In other embodiments, such an axial cavity 60 of the shaft 6 may be also be employed for other reasons as detailed below.

Various embodiments include features to facilitate spatial orientation of the valve V with respect to the implantation site. In various embodiments, the shaft 6 is flexible and adapted to be imparted specific curved shapes. The shaft 6 being flexible and selectively bendable makes it possible to deflect or "steer" the holder unit 10 with respect to the handle 1. Due to such deflectability or steerability the practitioner can select a desired spatial orientation of the holder unit 10 (and thus of the valve V) which facilitates positioning the valve V at the implantation site with a desired spatial orientation. This orientation may correspond to an orientation that avoids or minimizes the application of undesired mechanical stresses to the implantation site (i.e. to the heart tissues of the patient), while achieving the desired orientation of the valve V.

Steerability of the holder unit 10 permits a main axis X10 of the holder unit 10 to be arranged at a desired orientation which is generally skew or bent with respect to the axis X1 of the proximal portion of the device. The axis X1 essentially corresponds to the main axis of the handle 1 and the parts of the device adjacent thereto (i.e. the proximal sleeve 4 and the rotary actuation member 3). Figure 1 is exemplary of the main axis X10 of the holder portion 10 being steered (i.e. bent) to an angle a with respect to the axis X1.

It will likewise be appreciated that any desired "radial" or "polar" orientation of the axis X10 with respect to the axis X1 may be simply achieved by the practitioner by rotating the device 100, as a whole, around the axis X1, by rotating the handle 1 within the practitioner's hand.

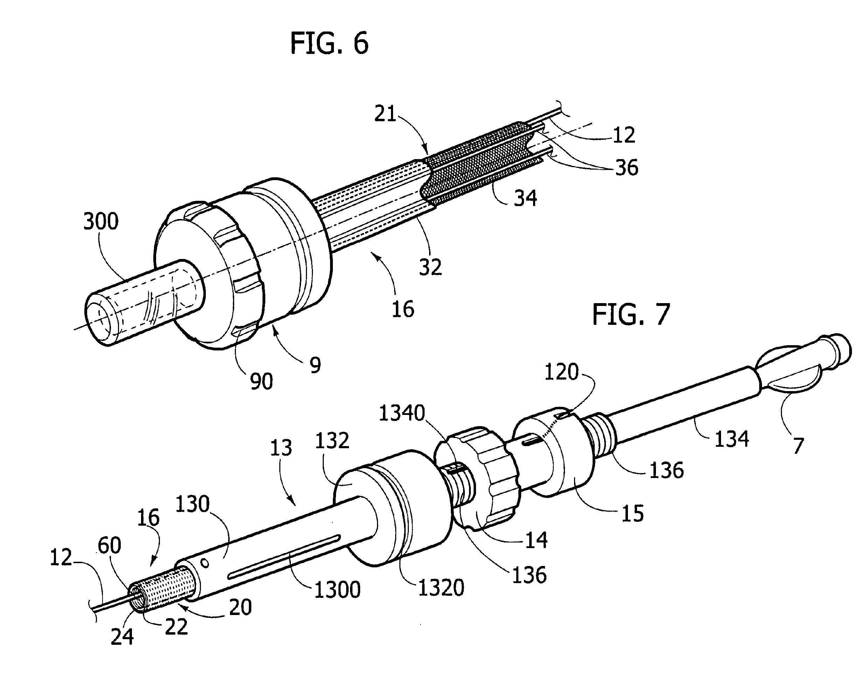

In various embodiments, the shaft 6 is made adjustable or "steerable" by means of a wire member 12 extending through the axial cavity 60 in the tubular core 16 and cooperating with a tensioning mechanism (see figure 7). In an embodiment, the tensioning mechanism includes a fixed tubular member 13, a rotary member 14 and an anchoring member 15.

The tubular member 13 includes a distal end 130 coupled and integral with a proximal end of the core 16, a radially expanded portion 132 and a proximal portion 134 provided with an outer thread 136. The distal end 130 thus forms a terminal abutment surface for the shaft 6.

The rotary element 14 is coupled to the outer thread 136 by means of an inner thread. The anchoring member 15 is slidably mounted over the outer thread 136 of the member 13 and is fixed in rotation (e.g., by means of a radial pin engaging a groove provided in the member 13).

The wire member 12 is anchored at the distal portion of the core 16 (e.g. in proximity of the inner body 9 carrying the annular portion 90 into which the portion V2 of the valve V is constrained) and extends within the shaft towards the mechanism 13, 14, 15.

With reference to figures 2a, 2b, in some embodiments, the actuation mechanism 13, 14, 15 extends through the proximal sleeve 4, the rotary actuation member 3 and the handle 1. The distal portion of the actuation mechanism may also extend partially into the sleeve 8 and the core 16. In some embodiments, the distal end 130 is inserted in the sleeve 4 with the radially expanded portion 132 providing an abutment surface to the sleeve 4.

Moreover, in various embodiments, the member 13 is provided with a longitudinal groove 1300 (see figure 7) adapted to rotationally fix the sleeve 4 with respect to the member 13 (e.g., by means of a radial pin or screw). In these embodiments, the length of the groove 1300 determines the longitudinal (i.e., axial) range of relative motion of the member 13 with respect to the sleeve 4. In other embodiments, the expanded portion 132 has an annular groove 1320, which is adapted to fix the rotating actuation member 3 in translation with respect to the member 13 (e.g., by means of a radial pin or screw engaging groove 1320), which allowing partial or complete rotational movement therein.

In various embodiments, the radially expanded portion 132, which is surrounded by the rotary actuation member 3 and the outer thread 136, as well as the whole proximal portion 134, is located inside the handle 1. In various embodiments, the member 13 has an elongated shape permitting it to extend within the handle 1 to be secured thereto (e.g., by means of radial screws), while also acting as a support member for the shaft 6. This ensures no rotation of the member 13 inside the device 100, since the handle 1 is firmly held by the practitioner's hand.

The mechanism 13, 14, 15 is intended to pull (i.e., to apply a longitudinal, tensile force to) the wire member 12 towards the handle 1 so that a longitudinal tensile force is applied to the core 16 to produce controlled bending of the shaft 6.

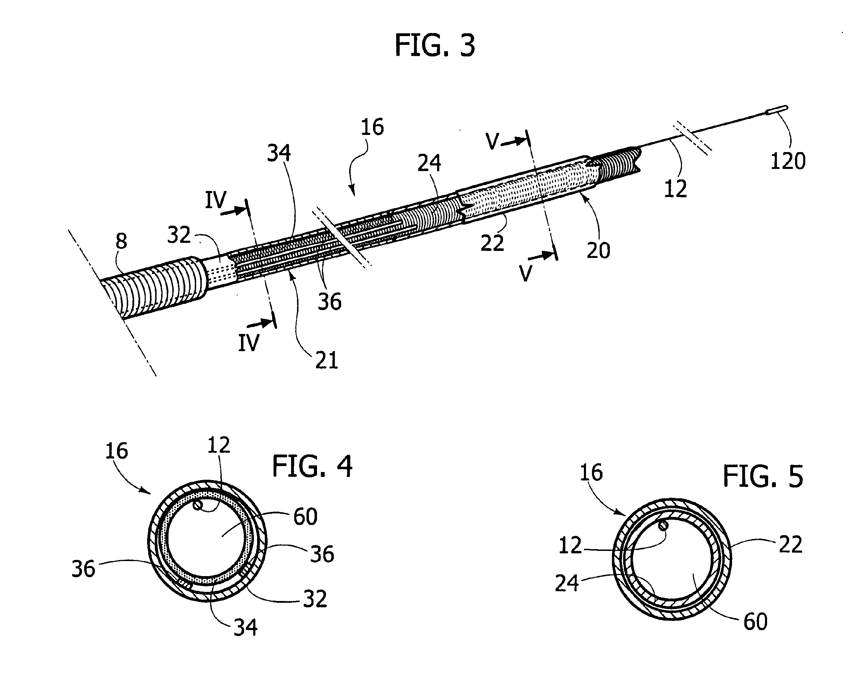

In various embodiments, the core 16 includes a proximal portion 20 and a distal portion 21. The proximal portion 20 (see, e.g., figure 3) includes an external sheath 22 and a coil element 24, helically wound therein. The coil element 24 is intended to provide a certain amount of flexibility to the core 16 (i.e., to the shaft 6), particularly to the proximal portion 20.

The distal portion 21 (see, e.g., figure 6) includes an external sheath 32 and a braided tubular element 34 located therein. A pair of longitudinal formations 36 is constrained between the external sheath 32 and the braided tubular element 34, and partially extends also between the coil element 24 and external sheath 22 of the proximal portion 20. In some embodiments, the longitudinal formations 36 are made of metallic material. The longitudinal formations 36 are intended to give a certain amount of stiffness to the distal portion 21 of the shaft 6, avoiding at the same time any undesired lateral bending thereof.

The coil element 24 and the braided tubular element 34 define an axial cavity, such as, for instance, the axial cavity 60, wherein the wire 12 extends from the distal portion of the core 16 to the member 15, where a proximal portion 120 of the wire member 12 is securely fixed.

In various embodiments, the wire 12 includes a proximal portion 120 which passes through a slot 1340 provided in the member 13 (see figure 7) and is anchored (for instance by mechanical clamping or crimping) to the member 15. In various embodiments, the wire 12 may be a tendon, a string, a suture, a wire, or a variety of other elements adapted to transmit a tensile force.

In various embodiments, the member 14 is a rotary ring-like member. Rotating the member 14 will thus cause the member 15 to slide axially relative to the member 1 in either direction depending on the direction the member 14 is rotated.

When rotated, the member 14 moves longitudinally in a proximal or distal direction, depending on the direction of rotation, along the outer thread 136 of member 13, thereby producing displacement of the member 15 over the member 13, proximally or distally depending on the direction of rotation of member 14.

In the case of a displacement of the member 14 in the proximal direction (i.e., towards or into the handle 1), the member 15 will be urged proximally to produce/increase longitudinal tensioning of the wire-like member 12, which, in turn, will translate into (increased) bending of the shaft 6.

In the case of a displacement of the member 14 in the distal direction (i.e., away or outwardly of the handle 1), the member 15 will correspondingly be able to slide distally thus releasing the tensile force on the wire-like element 12. This will gradually release its longitudinal tension, thereby reducing the amount of bending between the axes X10 and X1. The members 14, 15 will remain in contact with each other as long as there is a longitudinal tension in the wire-like element 12, acting as a sort of bias on members 14, 15. This ensures correspondence between the displacements of members 14 and 15 (i.e., smooth adjustment of the amount of bending). The amount of bending (i.e., the resulting angle α between the axes X10 and X1 in figure 1) can thus be selectively adjusted by the practitioner by acting on the rotary member 14.

In the embodiments considered herein the distal portion 21 of the tubular core 16 is intended to achieve the desired amount of bending with respect to the axis X1 having a minimum flexibility, while the proximal portion 20 is given a certain amount of flexibility substantially without being angularly displaced from the axis X1.

In various embodiments, the handle 1 is provided with an opening or window 140 through which the rotary member 14 can be actuated by the practitioner (e.g., by alternate action of the thumb). This exemplary mechanism provides the benefit of being actuatable by the practitioner by rotating the rotary member 14 while retaining a firm hold of the handle 1.

Rotation can be, as previously described, in either direction, so that the amount of longitudinal tension applied on the member 12 can be selectively varied while the bending angle of the shaft 6 will correspondingly vary based an the amount of tension applied by the member 12. The angle between the axes X10 and X1 (i.e. the spatial orientation of the holder portion 10 and the valve V located therein) can thus be selectively varied depending on the practitioner's needs and preferences during the intervention.

Those skilled in the art will appreciate that the action of applying a longitudinal tension onto the member 12 can be achieved by resorting to different mechanisms (e.g., by means of screw mechanism actuated by rotating the handle 1).

The embodiment of figures 8 and 9 may adopt, insofar as the release/delivery mechanism of the valve V is concerned, the same "micrometric" mechanism actuated via the rotary member 3 as discussed above. In the embodiments of figures 8 and 9, the desired "steering" of the holder portion 10, causing the angle X10 to form an adjustable angle α to the axis X1, can be achieved by coupling to the shaft a shaping member 5 (see figure 9) such as, for instance, a wire-like shaping member 5 inserted into an axial cavity of the shaft 6. In various embodiments, such a cavity may be the cavity 60 already provided for the fiber optic element 2000 to extend through the core 16 (as shown, e.g., in figure 2b).

In various embodiments, the shaping member 5 (figure 9) can be comprised of a bent steel rod rigid enough that, when inserted and advanced into the flexible shaft 6, the shaping member 5 will impart to the shaft 6 a bent shape which will correspond to the bent shape of the member. The composite shape of the bending member 5 and the flexible shaft 6 will depend on the bending resistance of each component.

In various embodiments, the shaping member 5 is one of an assortment of otherwise similar shaping member having different values for the "steering" angle α between X1 and X10 to be imparted to the shaft 6. Accordingly, once access to the implantation size is gained, the practitioner may evaluate the desired orientation of the holder portion 10 which will allow optimal delivery of the valve V at the implantation site. The practitioner will then select a positioning member 5 out of the assortment as the one providing such desired orientation. The shaping member thus selected will then be inserted into the shaft 6 to impart to the shaft the desired mutual orientation of the axes X10 to the axes X1.

Various modifications and additions can be made to the exemplary embodiments discussed without departing from the scope of the present invention. Accordingly, the scope of the present invention is intended to embrace all such alternatives, modifications, and variations as fall within the scope of the claims, together with all equivalents thereof.

## Claims

1. A device for delivering a cardiac valve prosthesis (V) to an implantation site, the device comprising a distal valve holder portion (10) and a shaft (6) coupled to the valve holder portion (10), wherein the shaft (6) is selectively bendable to a curved shape to selectively vary the spatial orientation of the valve holder portion (10) with respect to the implantation site, the device being **characterized in that** the shaft (6) comprises a tubular core (16) including a proximal portion (20) and a distal portion (21),
wherein the proximal portion (20) includes an external sheath (22) and a coil element (24) helically wound therein,
wherein the distal portion (21) includes an external sheath (32) and a braided tubular element (34) located therein, and
wherein a pair of longitudinal formations (36) is constrained between the external sheath (32) of the distal portion (21) and the braided tubular element (34) and partially extends also between the coil element (24) and the external sheath (22) of the proximal portion (20).

2. The device of claim 1, including a curved shaping member (5) for coupling to the shaft (6) to impart to the shaft (6) a curved shape corresponding to the curved shape of the shaping member (5).

3. The device of claim 2, wherein the shaft (6) has an axial cavity for insertion of the curved shaping member (5).

4. The device of either claim 2 or 3, wherein the curved shaping member (5) is in the form of a curved rod.

5. The device of any of claims 2 to 4, wherein the curved shaping member (5) for coupling to the shaft (6) is selectable out of a plurality of shaping members (5) each having a respective curved shape, whereby the shaft (6) is imparted different curved shapes by coupling it to different shaping members (5) in the assortment.

6. The device of claim 1, wherein the shaft (6) is configured to bend when subjected to a longitudinal force, preferably when subjected to a longitudinal tensile force.

7. The device of claim 6, wherein the shaft (6) has a cavity (60) and a wire-like member (12) extending in the cavity (60), the wire-like member (12) having associated tensioning members (13, 14, 15) to apply a longitudinal force to the wire-like member (12) to produce bending of the shaft (6).

8. The device of either of claim 6 or 7, including a rotary actuation member (14) adapted to apply a longitudinal force to the shaft (6).

9. The device of claim 8, including a handle (1) with the shaft (6) extending from the handle (1), the handle (1) including the rotary actuation member (14) located therein and open to access (140) from outside the handle (1).

10. The device of claim 9, including an opening (140) in the handle (1) for access to the rotary actuation member (14).

11. The device of any of claims 7 to 10, wherein the tensioning members (13, 14, 15) include:
a tubular member (13) having a terminal abutment surface (130) for the shaft (6);
an anchoring member (15) for anchoring a proximal end (120) of the wire-like member (12); and
an actuation member (14) actuatable between the tubular member (13) and the anchoring member (15), the actuation member (14) operable to selectively produce relative movement of the anchoring member (15) with respect to the tubular member (13) to thereby apply a longitudinal tensile force to the wire-like member (12).

12. The device of any of the previous claims, for deploying a cardiac valve prosthesis (V) including at least one radially expandable annular portion (V1, V2), wherein the valve holder portion (10) includes at least one constraint member (800) for radially constraining the at least one annular portion (V1, V2), the at least one constraint member (800) actuatable to release the at least one annular portion (V1, V2) constrained thereby to permit radial expansion thereof.

13. The device of claim 12, wherein the at least one constraint member (800) includes at least one sleeve (4, 8) slidably actuatable along the shaft (6), whereby the at least one constraint member (800) releases the at least one annular portion (V1, V2) constrained thereby.

14. The device of any of the previous claims, wherein the distal valve holder portion (10) has a diameter of between about 10mm and about 27mm.

15. The device of any of the previous claims, wherein the distal valve holder portion (10) has a marginal outer edge provided with at least one notch (802) providing reference in angular positioning of the cardiac valve prosthesis (V).

## Patentansprüche

1. Vorrichtung zum Verbringen einer Herzklappenprothese (V) an einen Implantationsort, mit einem distalen Klappenhalteabschnitt (10) und einem Schaft (6), der mit dem Klappenhalteabschnitt (10) verbunden ist, wobei der Schaft (6) wahlweise in eine gekrümmte Form biegbar ist, um die räumliche Orientierung des Klappenhalteabschnittes (10) in Bezug auf den Implantationsort nach Bedarf zu variieren, **dadurch gekennzeichnet, dass** der Schaft (6) einen röhrenförmigen Kern (16) aufweist, der einen proximalen Abschnitt (20) und einen distalen Abschnitt (21) umfasst,
wobei der proximale Abschnitt (20) eine äußere Umhüllung (22) und ein darin helixartig gewundenes Spiralelement (24) umfasst,
wobei der distale Abschnitt (21) eine äußere Umhüllung (32) und einen darin angeordnetes geflochtenes Röhrenelement (34) umfasst, und
wobei ein Paar länglicher Gebilde (36) zwischen der äußeren Umhüllung (32) des distalen Abschnittes (21) und dem geflochtenen Röhrenelement (34) eingeklemmt ist und sich teilweise auch zwischen dem Spiralelement (24) und der äußeren Umhüllung (22) des proximalen Abschnitts (20) erstreckt.

2. Vorrichtung nach Anspruch 1, mit einem gekrümmten Formungselement (5), das mit dem Schaft (6) verbindbar ist, um dem Schaft (6) eine gekrümmte Form zu verleihen, die der gekrümmten Form des Formungselementes (5) entspricht.

3. Vorrichtung nach Anspruch 2, **dadurch gekennzeichnet, dass** der Schaft (6) einen sich axial erstreckenden Hohlraum zum Einführen des gekrümmten Formungselementes (5) aufweist.

4. Vorrichtung nach einem der Ansprüche 2 oder 3, **dadurch gekennzeichnet, dass** das gekrümmte Formungselement (5) die Form eines geknickten Stabes hat.

5. Vorrichtung nach einem der Ansprüche 2 bis 4, **dadurch gekennzeichnet, dass** das mit dem Schaft (6) verbindbare gekrümmte Formungselement (5) aus einer Mehrzahl von Formungselementen (5) auswählbar ist, wobei jedes Formungselement (5) eine bestimmte gekrümmte Form derart besitzt, dass dem Schaft (6) verschiedene gekrümmte Formen verliehen werden, wenn er mit verschiedenen Formungselementen (5) aus dem Sortiment verbunden wird.

6. Vorrichtung nach Anspruch 1, **dadurch gekennzeichnet, dass** der Schaft (6) dazu ausgebildet ist, sich zu krümmen, wenn er mit einer längsgerichteten Kraft beaufschlagt wird, bevorzugt wenn er mit einer längsgerichteten Zugkraft beaufschlagt wird.

7. Vorrichtung nach Anspruch 6, **dadurch gekennzeichnet, dass** der Schaft (6) einen Hohlraum (60) und ein drahtförmiges Element (12) umfasst, das sich in den Hohlraum (60) erstreckt, wobei dem drahtförmigen Element (12) Zugelemente (13, 14, 15) zugeordnet sind, um das drahtförmige Element (12) zum Krümmen des Schafts (6) mit einer längsgerichteten Kraft zu beaufschlagen.

8. Vorrichtung nach einem der Ansprüche 6 oder 7, mit einem drehbaren Betätigungselement (14), das dazu ausgebildet ist, den Schaft (6) mit einer längsgerichteten Kraft zu beaufschlagen.

9. Vorrichtung nach Anspruch 8, mit einem Griff (1), von dem aus sich der Schaft (6) erstreckt, wobei der Griff (1) das drehbare Betätigungselement (14) umfasst, das darin von dem Außenbereich des Griffes (1) offen zugänglich angeordnet ist.

10. Vorrichtung nach Anspruch 9, mit einer Öffnung (140), die in dem Griff (1) für den Zugang zu dem drehbaren Betätigungselement (14) vorgesehen ist.

11. Vorrichtung nach einem der Ansprüche 7 bis 10, **dadurch gekennzeichnet, dass** die Zugelemente (13, 14, 15)
ein Röhrenelement (13) mit einer endständigen Anlagefläche (130) für den Schaft (6);
ein Verankerungselement (15) zum Verankern eines proximalen Endes (120) des drahtförmigen Elementes (12); und
ein Betätigungselement (14), das zwischen dem Röhrenelement (13) und dem Verankerungselement (15) betätigbar ist, wobei das Betätigungselement (14) betätigbar ist, um eine gewünschte relative Verschiebung des Verankerungselementes (15) gegenüber dem Röhrenelement (13) zu bewirken und dadurch das drahtförmige Element (12) mit einer längsgerichteten Zugkraft zu beaufschlagen;
umfassen.

12. Vorrichtung nach einem der vorhergehenden Ansprüche, zum Entfalten einer Herzklappenprothese (V), die wenigstens einen radial expandierbaren ringförmigen Abschnitt (V1, V2) aufweist, wobei der Klappenhalteabschnitt (10) wenigstens ein Presselement (800) umfasst, um den wenigstens einen ringförmigen Abschnitt (V1, V2) radial zusammenzupressen, wobei das wenigstens eine Presselement (800) betätigbar ist, um den wenigstens einen von ihm zusammengepressten ringförmigen Abschnitt (V1, V2) freizugeben, um ihm ein radiales Expandieren zu erlauben.

13. Vorrichtung nach Anspruch 12, **dadurch gekennzeichnet, dass** das wenigstens eine Presselement (800) wenigstens eine Hülse (4, 8) umfasst, die durch Verschieben entlang des Schaftes (6) betätigbar ist, wobei das wenigstens eine Presselement (800) den wenigstens einen von ihm zusammengepressten ringförmigen Abschnitt (V1, V2) freigibt.

14. Vorrichtung nach einem der vorhergehenden Ansprüche, **dadurch gekennzeichnet, dass** die distale Klappenhalteabschnitt (10) einen Durchmesser zwischen etwa 10 mm und etwa 27 mm besitzt.

15. Vorrichtung nach einem der vorhergehenden Ansprüche, **dadurch gekennzeichnet, dass** der distale Klappenhalteabschnitt (10) am Rand eine Außenkante aufweist, die mit wenigstens einer Kerbe (802) versehen ist, die bei einem Winkelpositionieren der Herzklappenprothese (V) als ein Bezugspunkt dient.

## Revendications

1. Dispositif permettant de mettre en place une prothèse de valvule cardiaque (V) dans un site d'implantation, le dispositif comprenant une partie distale de maintien de valvule (10) et une tige (6) couplée à la partie de maintien de valvule (10), dans lequel la tige (6) peut sélectivement être fléchie de manière à avoir une forme incurvée pour faire varier sélectivement l'orientation spatiale de la partie de maintien de valvule (10) par rapport au site d'implantation, le dispositif étant **caractérisé en ce que** la tige (6) comprend un noyau tubulaire (16) comportant une partie proximale (20) et une partie distale (21),
dans lequel la partie proximale (20) comporte une gaine externe (22) et un élément de bobine (24) enroulé de manière hélicoïdale dans celle-ci,
dans lequel la partie distale (21) comporte une gaine externe (32) et un élément tubulaire tressé (34) situé dans celle-ci, et
dans lequel une paire de formations longitudinales (36) est contrainte entre la gaine externe (32) de la partie distale (21) et l'élément tubulaire tressé (34) et s'étend partiellement aussi entre l'élément de bobine (24) et la gaine externe (22) de la partie proximale (20).

2. Dispositif de la revendication 1, comportant un élément de mise en forme incurvé (5) pour se coupler à la tige (6) afin de conférer à la tige (6) une forme incurvée correspondant à la forme incurvée de l'élément de mise en forme (5).

3. Dispositif de la revendication 2, dans lequel la tige (6) a une cavité axiale pour l'insertion de l'élément de mise en forme incurvé (5).

4. Dispositif de l'une ou l'autre des revendications 2 et 3, dans lequel l'élément de mise en forme incurvé (5) se présente sous la forme d'une barre incurvée.

5. Dispositif de l'une des revendications 2 à 4, dans lequel l'élément de mise en forme incurvé (5) pour se coupler à la tige (6) peut être sélectionné parmi une pluralité d'éléments de mise en forme (5) ayant chacun une forme incurvée respective, moyennant quoi différentes formes incurvées sont conférées à la tige (6) en la couplant à différents éléments de mise en forme (5) dans l'assortiment.

6. Dispositif de la revendication 1, dans lequel la tige (6) est configurée de manière à se fléchir lorsqu'elle est soumise à une force longitudinale, de préférence lorsqu'elle est soumise à une force de traction longitudinale.

7. Dispositif de la revendication 6, dans lequel la tige (6) a une cavité (60) et un élément de type fil (12) s'étendant dans la cavité (60), l'élément de type fil (12) ayant des éléments de tension associés (13, 14, 15) pour appliquer une force longitudinale à l'élément de type fil (12) afin de produire une flexion de la tige (6).

8. Dispositif de l'une ou l'autre des revendications 6 ou 7, comportant un élément d'actionnement rotatif (14) adapté pour appliquer une force longitudinale à la tige (6).

9. Dispositif de la revendication 8, comportant une poignée (1) avec la tige (6) s'étendant à partir de la poignée (1), la poignée (1) comportant l'élément d'actionnement rotatif (14) situé dans celle-ci et s'ouvre pour un accès (140) depuis l'extérieur de la poignée (1).

10. Dispositif de la revendication 9, comportant une ouverture (140) dans la poignée (1) permettant un accès à l'élément d'actionnement rotatif (14).

11. Dispositif de l'une des revendications 7 à 10, dans lequel les éléments de tension (13, 14, 15) comportent :
un élément tubulaire (13) ayant une surface de butée terminale (130) pour la tige (6) ;
un élément d'ancrage (15) pour ancrer une extrémité proximale (120) de l'élément de type fil (12) ; et
un élément d'actionnement (14) pouvant être actionné entre l'élément tubulaire (13) et l'élément d'ancrage (15), l'élément d'actionnement (14) pouvant fonctionner pour produire sélectivement un mouvement relatif de l'élément d'ancrage (15) par rapport à l'élément tubulaire (13) afin d'appliquer ainsi une force de traction longitudinale à l'élément de type fil (12).

12. Dispositif de l'une des revendications précédentes, permettant de déployer une prothèse de valvule cardiaque (V) comportant au moins une partie annulaire radialement extensible (V1, V2), où la partie de maintien de valvule (10) comporte au moins un élément de contrainte (800) pour contraindre radialement l'au moins une partie annulaire (V1, V2), l'au moins un élément de contrainte (800) pouvant être actionné pour libérer l'au moins une partie annulaire (V1, V2) ainsi contrainte afin de permettre une expansion radiale de celle-ci.

13. Dispositif de la revendication 12, dans lequel l'au moins un élément de contrainte (800) comporte au moins un manchon (4, 8) pouvant être actionné de manière coulissante le long de la tige (6), moyennant quoi l'au moins un élément de contrainte (800) libère l'au moins une partie annulaire (V1, V2) ainsi contrainte.

14. Dispositif de l'une des revendications précédentes, dans lequel la partie distale de maintien de valvule (10) a un diamètre compris entre environ 10 mm et environ 27 mm.

15. Dispositif de l'une des revendications précédentes, dans lequel la partie distale de maintien de valvule (10) a un bord externe marginal doté d'au moins une encoche (802) fournissant une référence dans le positionnement angulaire de la prothèse de valvule cardiaque (V).
